# EUROPEAN PATENT SPECIFICATION

(11) **EP 2 029 032 B1**
(45) Date of publication and mention of the grant of the patent: **26.02.2014**
(21) Application number: 07733673.3
(22) Date of filing: 10.05.2007
(51) Int. Cl.: A61B 17/10, A61B 17/00, A61B 17/08

(54) **HEART VALVE REPAIR**
HERZKLAPPENREAPARATUR
REPARATION D'UNE VALVE CARDIAQUE

(30) Priority: 10.05.2006 GB 0609151
(43) Date of publication of application: 04.03.2009
(73) Proprietor: St. Jude Medical, Cardiology Division, Inc., St. Paul MN 55117 (US)
(72) Inventor: Wells, Francis, Cambridge, CB3 8RE (GB); Schofield, Peter Marshall, Cambridge, CB3 8RE (GB)
(74) Representative: Fleck, Barbara
(86) International application number: PCT/GB2007/050252
(87) International publication number: WO 2007/129125

(56) References cited:
- WO-A-99/00059
- WO-A1-01/28432
- US-A- 5 156 608
- US-A- 5 431 669
- US-A1- 2005 240 202
- US-B1- 6 945 978

## Description

### Field of invention

The invention relates to a system for repairing a heart valve. In particular, the invention relates to systems specifically adapted to repair a mitral heart valve wherein one leaflet has a prolapsed segment.

### Background of invention

The heart valves are valves in the heart that maintain the unidirectional flow of blood by opening and closing, depending on the difference in pressure on each side. There are four valves of the heart. The two atrioventricular valves (mitral and tricuspid valves) are large, multicusped valves that prevent backflow from the ventricles into the atria during systole. They are anchored to the wall of the ventricle by chordae tendinae, which prevent the valve from inverting. The two semilunar valves are positioned on the pulmonary artery and the aorta and prevent blood flowing back from the arteries into the ventricles.

The mitral valve is situated at the gate of the left ventricle and is made up of two leaflets (flaps) and a diaphanous incomplete ring around the valve, known as the mitral valve annulus. When the valve opens, blood flows into the left ventricle. After the left ventricle fills with blood and contracts, the two leaflets of the mitral valve are pushed upwards and close, permitting no blood to flow back into the left upper chamber (atrium) and the lungs.

Conditions associated with a dysfunction of the mitral valve can be divided into stenosis (the valve does not open properly) and regurgitation (the closure of the valve is affected).

Mitral valve prolapse (MVP) is a type of myxomatous valve disease in which the abnormal mitral valve leaflets prolapse (i.e. billowed, loose and floppy). Furthermore, the chordae tendinea stretch and thus become too long. As a result, the valve does not close normally. As a result of being stretched, the unsupported valve leaflet bulges back, or "prolapses," into the left atrium like a parachute. Thus, as the ventricle contracts the abnormal leaflet is propelled backwards, beyond its normal closure line into the left atrium allowing blood to return back into the left atrium and the lungs. This produces a systolic mitral regurgitation murmur.

Mitral valve prolapse may also be associated with Marfan's syndrome and other connective tissue disorders.

MVP causes mitral regurgitation (MR). Isolated posterior leaflet prolapse of the human heart mitral valve, i.e. prolapse of a single leaflet, is the most common cause of MR. The exact cause of the prolapse is not clear. Untreated MR will lead to congestive heart failure, and pulmonary hypertension (high blood pressure in the lung).

Current methods to treat MVP include resection of the prolapsed segment using open heart surgery or replacement of the mitral valve. Another method, edge-to-edge or bow-tie technique, is also used (Alfieri et al, The Journal of Thoracic and Cardiovascular Surgery, 2001, Vol. 122, pages 674-688). Research is in progress to allow this to be carried out percutaneously. In this method, both of the two mitral valve leaflets are grasped together and clipped to each other. Thus, the technique is designed to be applied to a prolapse of both leaflets. This method has really only reached the stage of proof of principle.

WO 99/00059 relates to a method for repairing the mitral heart valve wherein the two leaflets are grasped together using forceps which enable the grasping and bringing together of both leaflets. The two leaflets are subsequently joined and secured by applying a fastening means.

WO 2004/082523 also relates to a method for repairing the mitral heart valve using the bow-tie technique. In this method, the two leaflets are sutured to each other, thus the leaflets are joined other at their mid point.

The disadvantage of the open surgical method is its invasive nature. The discomfort and recovery period for the patient means a 6-10 day stay in hospital. Furthermore, the Alfieri technique results in a non-physiological orifice to the mitral value giving rise to a double orifice.

US-A-2005/0240202 discloses a device to capture two leaflets and to opposition the two adjacent leaflets along their edges. The book of the engagement member is not configured to invert a single prolapsed heart value leaflet.

There exists a need for an alternative treatment of single heart valve leaflet prolapse, reducing the trauma of the patient, and giving a normal physiological result.

### Description of the invention

The invention relates to a system for repairing a heart valve having an engagement member and a fastener.

According to a first aspect of the present invention, there is provided a system for repairing a single prolapsed leaflet of a heart valve comprising a catheter, said catheter comprising an engagement member, the engagement member having at its distal end a means capable of engaging with the surface of a heart valve leaflet; and a fastener capable of deploying a fastening means to said leaflet.

By 'distal' is meant the portion of the engagement member nearest the prolapsed leaflet of a patient, while 'proximal' means the portion of the engagement member furthest from the prolapsed leaflet.

The invention relates to a device that is capable of repairing a heart valve with a single prolapsed segment. Accordingly, the system is particularly adapted so that it has an engagement member that is capable of grasping /engaging with a single leaflet that is prolapsed.

The device has a single engagement member. For example, the device may be as shown in the figures. As shown in the figures, the device has a single engagement member that has at its distal end a hook which is capable of engaging with a single leaflet. Due to the presence of a single engagement member, grasping a single prolapsed leaflet is facilitated.

The system of the invention may be inserted into the left ventricle of the heart via the femoral artery in the groin. Through one lumen of the catheter, an engagement member having a hook at its distal end will be passed. This hook engages with or catches the prolapsed portion of a heart valve leaflet. The engagement member is movable and can be pulled towards the interior of the left ventricle, thereby pulling down the prolapsed portion of the leaflet. Due to this pulling action, the prolapsed portion inverts. The leaflet will be drawn down until it is in the correct position to re-align the leaflet having a prolapsed segment and the normal leaflet in normal coaptation.

When the correct alignment has been achieved, a fastener is introduced through a second channel within the catheter. The fastener is movable. The fastener is capable of delivering one or more fastening means to the inverted segment in order to clip together the now inverted edges of the previously prolapsing portion of the leaflet. Thus, the inverted excess tissue is secured, thereby preventing the excess tissue from flapping and also preventing the further prolapse of the segment. The heart valve is thus capable of closing in normal coaptation and regurgitation is prevented.

The present invention allows the treatment of isolated heart valve prolapse, as the system of the invention is specifically designed to allow engagement of the engagement member with the surface of a single heart valve leaflet. As the system is introduced using a catheter, there is no requirement for open heart surgery and thus trauma for the patient is reduced.

The engagement member is capable of engaging with the surface of a heart valve leaflet, preferably with the prolapsed segment of a leaflet. Preferably, the engagement member is capable of inverting said leaflet. Also preferably, the engagement member is further capable of aligning the leaflet with the second (i.e. opposing) leaflet in normal coaptation/alignment.

Preferably, the heart valve is the mitral heart valve. In one embodiment, the leaflet is the posterior leaflet. In another embodiment, the leaflet is the anterior leaflet.

In one embodiment of the invention, the heart valve is the tricuspid valve.

Preferably, the means at the distal end of the engagement member is a hook. For example, the engagement member is a hooked wire.

The fastening means is preferably in the form of one or more clips to avoid the need for sutures. However, in an alternative embodiment, the fastening means may be a staple, coil, cufflink-link fastener, suture or other fasteners known in the art.

Preferably, the fastening means is made of a biocompatible material, i.e. material that the body generally accepts without a major immune response. Biocompatible materials useful in the invention are known in the art, including medical-grade polymers, metals, ceramics, pyrolytic carbon, composites and natural materials. Examples of biomaterials are poly(esters) based on polylactide, polyglycolide, polycaprolactone, and their copolymers, poly(hydroxyalkanoate)s of the PHB-PHV class, polyvinylchloride, polytetrafluoroethylene, nylon, polyethylene oxide, polyethylene glycol, ethylene-vinyl acetate copolymer, copolymers of polyethylene oxide and polybutylene terephthalate, silicon; natural polymers, particularly, modified poly(saccharide)s, e.g., starch, cellulose or chitosan.

In one embodiment, the fastening means is made of shape memory material. A shape-memory material is one that undergoes a change of form at a certain temperature called the transformation temperature. Above this temperature, the material has one form and below this temperature it has another form. The low temperature structure of these types of materials allows the material to be easily deformed. However, on heating the material returns to its high temperature structure. Thus, a shape memory material can undergo substantial plastic deformation, and then be triggered into returning to its original shape by the application of heat.

The fastening means according to the invention may thus comprise shape memory material having a small cross-section when introduced into a body and heart. After it is released from the fastener and deployed to the leaflet, the fastening means is triggered by heat from the body and will return to its original "memorised" shape. A number of suitable shape memory materials are known in the art and can be used according to the invention, including titanium-palladium-nickel, nickel-titanium-copper, gold-cadmium, iron-zinc-copper-aluminium, titanium-niobium-aluminium, uranium-niobium, hafnium-titanium-nickel, Iron-manganese-silicon, nickel-titanium (e.g. nitinol®), nickel-iron-zinc-aluminium, copper-aluminium-iron, titanium-niobium, zirconium-copper-zinc, nickel-zirconium-titanium.

The method for repairing a heart valve comprising engaging the prolapsed portion of a single leaflet is carried out using a catheter.

The fastener according to the methods invention may be a single device operating in concert with engagement member. Alternatively, the fastener may be separate device which is independent from the grasper. The engagement member and fastener may be housed in a single catheter or separate catheters.

The catheter(s) may be inserted through the femoral artery.

### Brief description of drawings

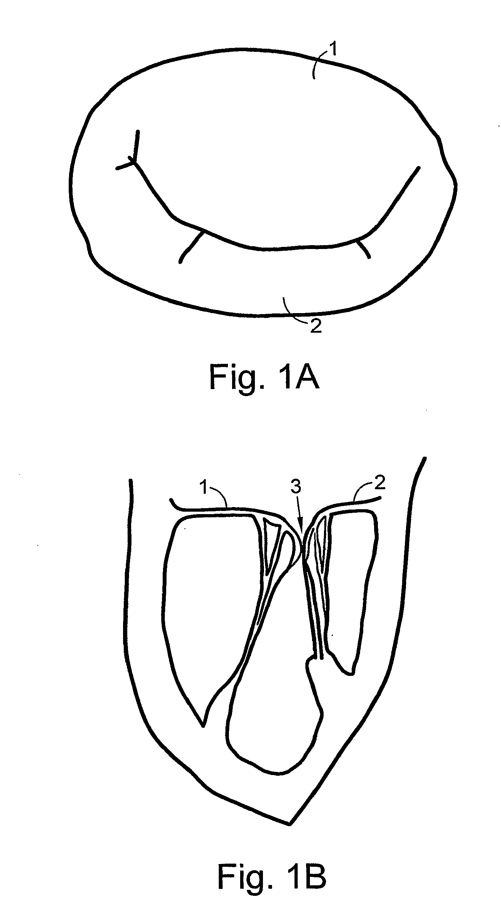

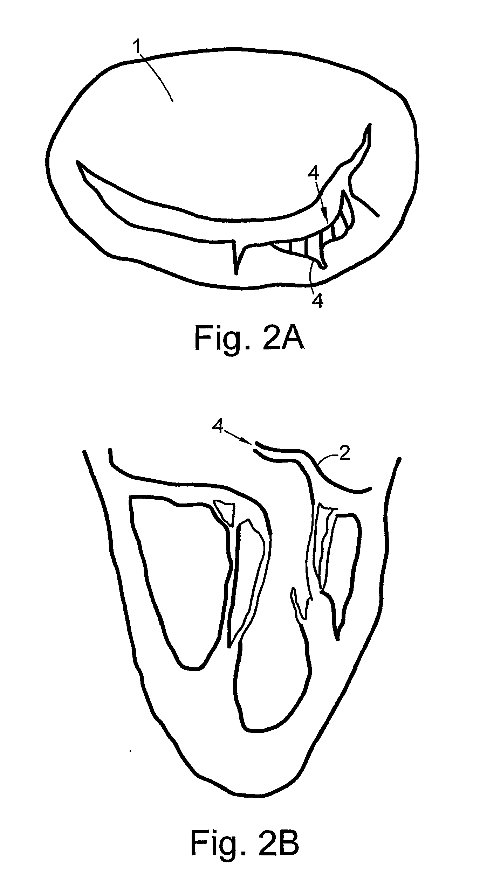

These and other aspects of the present invention will now be described by way of example only and without limitation with reference to the accompanying drawings in which Figure 1 shows normal mitral heart valve leaflets. In Fig. 2, the posterior leaflet has an isolated prolapsed segment. Thus, normal coaptation cannot be achieved.

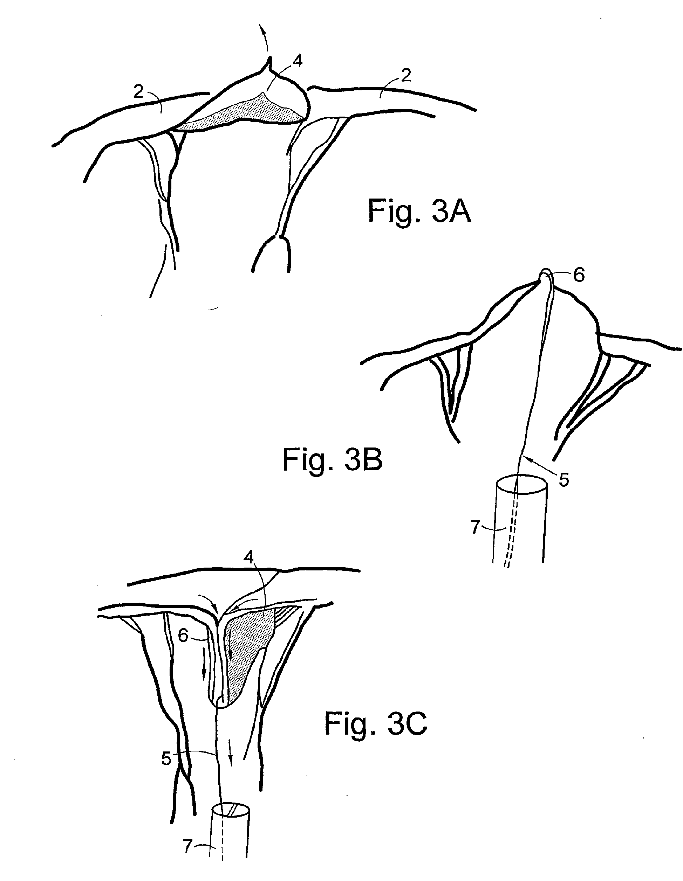

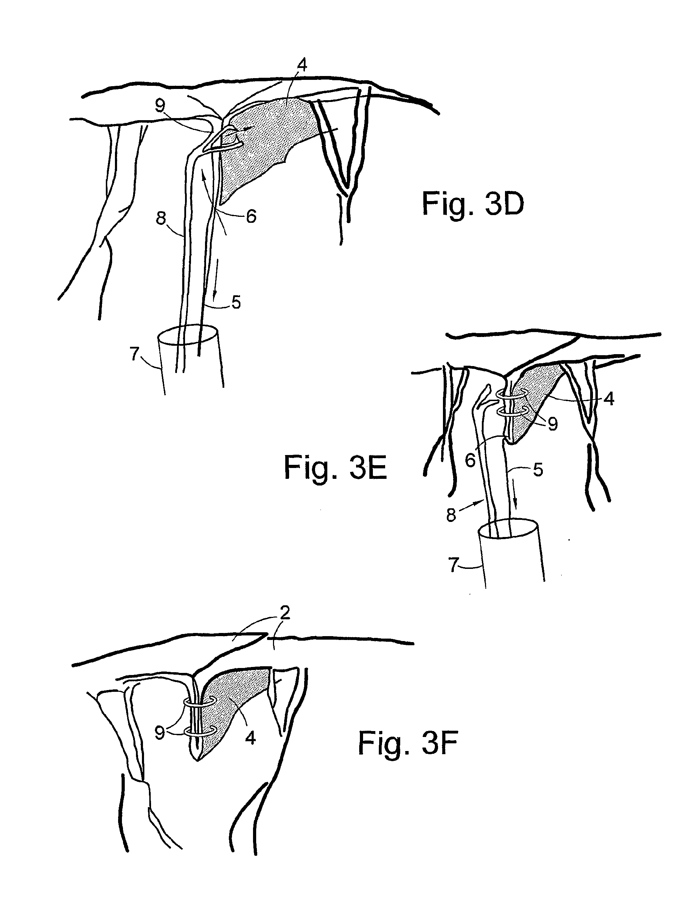

Figure 3 shows the steps for method for repairing a regurgitant heart valve according to the invention, using a device as described herein.

### Detailed description of drawings

Figure 1A shows a transverse section normal mitral heart valve leaflets. Figure 1B is a cross section of the leaflet ventricle of the heart, showing a normal anterior (1) and a normal posterior (2) leaflet when the mitral valve is closed. The mitral valve shown in Figure 1B closes properly; the leaflets are joined in normal coaptation (3). Under normal conditions, the coaptation point of the mitral valve leaflets in systole practically reaches the level of the mitral annulus.

This point is displaced into the atrium in abnormal conditions, such as morphological abnormalities of the leaflets or dilatation of the left ventricle. As a result, the distance between the coaptation point of the leaflets and the level of the mitral annulus is increased.

Figure 2 shows an abnormal posterior leaflet (2) with a prolapsed segment (4) as an transverse section (2A) and as part of the mitral valve in the heart as a cross section (2B). The prolapsed segment of the posterior leaflet (4) prevents the valve from closing properly as the excess tissue is now unsupported as a result of chordal elongation and/or rupture, and does not join with the second leaflet for proper closure of the valve.

Figure 3 demonstrates the device of the invention. Figure 3A shows the posterior leaflet (2) with an isolated prolapsed segment (4). In figure 3B, a catheter (7) is shown having an engagement member (5) having a hook (6) at it's distal end, i.e. the end furthest away from the catheter and closest to the prolapsed portion of the leaflet. The hook (6) is capable of engaging with the atrial surface of the isolated prolapsed segment of the leaflet (4). The engagement member is capable of pulling the segment inside the left ventricle, thereby inverting the prolapsed segment of the leaflet, i.e. inverting the excess tissue.

In Figure 3C, the excess tissue (4) is inverted and kept in place or stabilised due to the engagement member (5), as the hook (6) remains engaged with the prolapsed portion (4).

In figure 3D, a fastener (8) is housed in the catheter (7) and is used to deploy a fastening means (9) to the inverted prolapsed segment (4). At the same time, the engagement member (5) remains engaged with the prolapsed portion (4), thus retaining the prolapsed portion (4) in place. This is achieved due to a pulling action of the engagement member (5), which is indicated with an arrow in figure 3D.

In Figure 3E, one, two or more separate fastening means (9) are now in place, securing the prolapsed segment (4). The fastener (8) is withdrawn from the prolapsed portion. The engagement member (5) can now be withdrawn.

Figure 3F shows the inverted and secured prolapsed segment (4) of the posterior leaflet (2), secured with two separate clips (9). The catheter (7) having an engagement member (5) and a fastener (8) has been removed.

## Claims

1. A system for repairing a single prolapsed leaflet of a heart valve comprising a catheter, said catheter (7) comprising a single engagement member (5), the engagement member (5) having at its distal end a hook (6) capable of engaging with the surface of a single prolapsed heart valve leaflet and capable of inverting said single prolapsed leaflet; and a fastener (8) capable of deploying a fastening means (9) to said single inverted leaflet.

2. A system according to claim 1 wherein the engagement member (5) is capable of aligning said leaflet with the second leaflet in normal coaptation.

3. system according to a preceding claim wherein the fastening means (8) is made of a biocompatible material.

4. system according to a preceding claim wherein the fastening means (8) is made of a shape memory material.

5. system according to a preceding claim wherein the fastening means is a clip (9).

## Patentansprüche

1. System zum Reparieren eines einzelnen vorgefallenen Segels einer Herzklappe, wobei das System einen Katheter umfasst, wobei der Katheter (7) ein einzelnes Eingriffselement (5) umfasst, wobei das Eingriffselement (5) an seinem distalen Ende einen Haken (6) hat, der dazu in der Lage ist, mit der Oberfläche eines einzelnen vorgefallenen Segels ineinanderzugreifen, und dazu in der Lage ist, das einzelne vorgefallene Segel umzuwenden, und ein Befestigungselement (8), das dazu in der Lage ist, ein Befestigungsmittel (9) an dem einzelnen umgewendeten Segel zu entfalten.

2. System nach Anspruch 1, wobei das Eingriffselement (5) dazu in der Lage ist, das Segel in einer normalen Koaptation mit dem zweiten Segel in Eingriff zu bringen.

3. System nach einem der vorhergehenden Ansprüche, wobei das Befestigungsmittel (9) aus einem biologisch verträglichen Werkstoff hergestellt ist.

4. System nach einem der vorhergehenden Ansprüche, wobei das Befestigungsmittel (9) aus einem Formgedächtnis-Werkstoff hergestellt ist.

5. System nach einem der vorhergehenden Ansprüche, wobei das Befestigungsmittel eine Klammer (9) ist.

## Revendications

1. Système de réparation d'une valve prolabée individuelle d'une valvule cardiaque, comprenant un cathéter, ledit cathéter (7) comprenant un seul élément d'engagement (5), l'élément d'engagement (5) comportant au niveau de son extrémité distale un crochet (6), capable de s'engager dans la surface d'une valvule cardiaque prolabée individuelle et capable d'inverser ladite valve prolabée individuelle ; et un élément de fixation (8), capable de déployer un moyen de fixation (9) vers ladite valve inversée individuelle.

2. Système selon la revendication 1, dans lequel l'élément d'engagement (5) est capable d'aligner ladite valve avec la deuxième valve en coaptation normale.

3. Système selon une revendication précédente, dans lequel le moyen de fixation (9) est composé d'un matériau biocompatible.

4. Système selon une revendication précédente, dans lequel le moyen de fixation (9) est composé d'un matériau à mémoire de forme.

5. Système selon une revendication précédente, dans lequel le moyen de fixation est une agrafe (9).
